# FASCICULE DE BREVET EUROPEEN

(11) **EP 0 889 026 B1**
(45) Date de publication et mention de la délivrance du brevet: **14.11.2001**
(21) Numéro de dépôt: 98401616.2
(22) Date de dépôt: 29.06.1998
(51) Int. Cl.: C07C 205/37, C07C 211/52

(54) **Apha-phenoxy-alcanols, leurs procédés de préparation et leurs applications**
Alpha-Phenoxyalkanole, Verfahren zu ihrer Herstellung und ihre Verwendungen
Alpha-phenoxy alkanols, processes for their preparation and their uses

(30) Priorité: 02.07.1997 FR 9708361
(43) Date de publication de la demande: 07.01.1999
(73) Titulaire: CFPI NUFARM, 92233 Gennevilliers (FR)
(72) Inventeur: Schapira, Joseph, 75015 Paris (FR); Cheminaud, Jean-Claude, 95220 Herblay (FR); Gasse, Jean-Jaques, 27600 Gaillon (FR); Schanen, Vincent, 75019 Paris (FR); Rondot, Benoit, 92300 Levallois Perret (FR); Lemoine, Jean-Claude, 92290 Chatenay (FR)
(74) Mandataire: Koch, Gustave

(56) Documents cités:
- EP-A- 0 630 883

## Description

L'invention a pour objet de nouveaux α-phénoxyalcanols.

Elle vise également un procédé de préparation de ces nouveaux α-phénoxy-alcanols ainsi que leurs applications comme intermédiaires de synthèse, notamment pour la préparation de la pendiméthaline qui est un herbicide sélectif appartenant à la classe des 2,6-dinitroanilines.

Il est connu de préparer la pendiméthaline par mise en oeuvre du procédé décrit dans le document EP 0630883.

La présente invention permet d'éviter les inconvénients du procédé connu grâce à l'utilisation des nouveaux α-phénoxy-alcanols, comme intermédiaire de synthèse pour la préparation de la pendiméthaline.

Les nouveaux α-phénoxy-alcanols conformes à l'invention ainsi que, lorsqu'ils comportent au moins un carbone asymétrique, leur stéroisomères de conformation R,S sont représentés par la formule générale 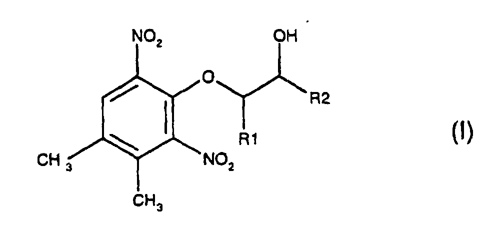 dans laquelle R₁ et R₂, qui sont identiques ou différents l'un de l'autre, représentent un atome d'hydrogène ou le radical CH₃.

Le procédé conforme à l'invention pour la préparation des α-phénoxy-alcanols de formule (I) est caractérisé par le fait que l'on fait réagir le 2,6-dinitro-3,4-diméthylphénol, avec un oxiranne inférieur choisi dans le groupe comprenant l'oxyde d'éthylène, l'oxyde propylène et le 2,3-époxybutane.

Le 2,6-dinitro-3,4-diméthylphénol peut être obtenu par toute technique de nitration connue à partir du 3,4-diméthylphénol qui est un produit du commerce.

C'est à partir du 3,4-diméthyl-phénoxy-alcanol de formule (I) susceptible d'être obtenu comme indiqué plus haut que l'on prépare la pendiméthaline.

Pour ce faire,
- soit on soumet le 2,6-dinitro-3,4-diméthyl-phénoxy-alcanol à une réaction d'amination, notamment à l'aide du 3-amino-pentane,
- soit on soumet le 3,4-diméthyl-phénoxy-alcanol à une réaction de nitration suivie d'une réaction d'amination du dérivé nitré ainsi formé, notamment à l'aide du 3-amino-pentane.

L'invention sera encore mieux comprise à l'aide du complément de description qui suit et des exemples non limitatifs relatifs à des modes de réalisation avantageux.

Les α-phénoxy-alcanols de formule (I) sont préparés en procédant à la réaction d'addition d'un oxiranne inférieur sur le 2,6-dinitro-3,4-diméthylphénol.

Cette réaction d'addition peut être mise en oeuvre au sein d'un solvant organique inerte.

Le solvant inerte en question est avantageusement choisi dans le groupe comprenant les essences aliphatiques dont notamment l'hexane, l'heptane, l'octane, le nonane et le dodécane, les dérivés chlorés dont notamment le dichlorométhane, le 1,2-dichloroéthane, le chloroforme, le trichloréthylène et le tétrachloréthylène, les dialkyl-éthers dont notamment les diéthyl- et dipropyl-éthers ainsi que les dibutyl-éthers, et de préférence les solvants aromatiques dont notamment le benzène, le toluène et le xylène.

La quantité de solvant mise en oeuvre lors de la réaction n'est pas critique.

De préférence, la réaction d'addition est conduite en l'absence de solvant, ce grâce à quoi une productivité accrue est obtenue.

La réaction d'addition est conduite à une température choisie dans le domaine de -40 à 300°C, de préférence de -20 à 200°C et plus préférentiellement encore de -10 à 150°C.

Elle peut être conduite à la pression atmosphérique ou sous pression autogène d'oxiranne.

De préférence, elle est mise en oeuvre sous pression partielle d'oxiranne contrôlée par l'introduction d'un gaz inerte, et plus particulièrement sous pression contrôlée d'argon ou d'azote.

La réaction est, de préférence, conduite sous une pression totale de 0,98692 à 98,692 bars, plus préférentiellement de 0,98692 à 24,67 bars et, plus préférentiellement encore, sous une pression totale inférieure à 9,8692 bars.

Les quantités molaires d'oxiranne mises en oeuvre par rapport au phénol engagé sont dans des rapports de 1 à 100, de préférence de 1 à 20 et, plus préférentiellement encore, de 1 à 5.

De préférence, on utilise une quantité d'oxiranne de 1 à 10 mol/mol de phénol, plus préférentiellement de 1 à 2 mol/mol et, plus préférentiellement encore, de 1 à 1,2 mol/mol.

La réaction peut être conduite en l'absence de catalyseur ou en présence de catalyseurs acides; de préférence, il est fait recours à un catalyseur basique choisi dans le groupe comprenant les amines tertiaires aliphatiques notamment du groupe comprenant la triméthylamine, la méthyldiéthylamine et la triéthylamine, les anilines tertiaires dont notamment la diméthylaniline et la diéthylaniline, les amines cycliques dont notamment la pyridine et les imidazoles; il est également possible d'avoir recours aux bases alcalines dont notamment la soude, la potasse et la chaux.

Le catalyseur est mis en oeuvre en des quantités catalytiques, c'est-à-dire des quantités en poids par rapport au poids de phénol engagé de 10 ppm à 10000 ppm et, de préférence, de 50 à 1000 ppm.

Les conditions de la réaction d'addition sont maintenues pendant un temps nécessaire et suffisant pour atteindre un taux de conversion du phénol supérieur à 99%; lorsqu'un degré de pureté plus élevé est exigé, la durée de réaction peut être prolongée en conséquence sans provoquer d'altération de la qualité de l'α-phénoxy-alcanol formé.

Les α-phénoxy-alcanols ainsi obtenus présentent une pureté supérieure à 99%, nécessaire et suffisante pour permettre la préparation dans l'utilisation conforme à l'invention d'une pendiméthaline technique de pureté supérieure à 97% exempte de nitrosamines; dans cette préparation de la pendiméthaline, toute technique de substitution nucléophile connue peut être utilisée.

Les α-phénoxy-alcanols conformes à l'invention et représentés par la formule (I) peuvent donc être facilement et avantageusement synthétisés à partir de dérivés phénoliques commerciaux ou facilement accessibles.

Ces α-phénoxy-alcanols sont des intermédiaires importants pour la synthèse économique et avantageuse de l'herbicide sélectif constitué par la pendiméthaline.

Une autre application de ces α-phénoxy-alcanols réside dans le fait qu'ils permettent la préparation des 2,6-dinitro-3,4-diméthylanilines qui sont des produits intermédiaires de synthèse importants, par réaction avec de l'ammoniaque, une amine primaire ou une amine secondaire.

La préparation de l'α-2,6-dinitro-3,4-diméthyl-phénoxy-propanol et son utilisation pour la préparation de la pendiméthaline peuvent être représentées par les schémas réactionnels suivants:

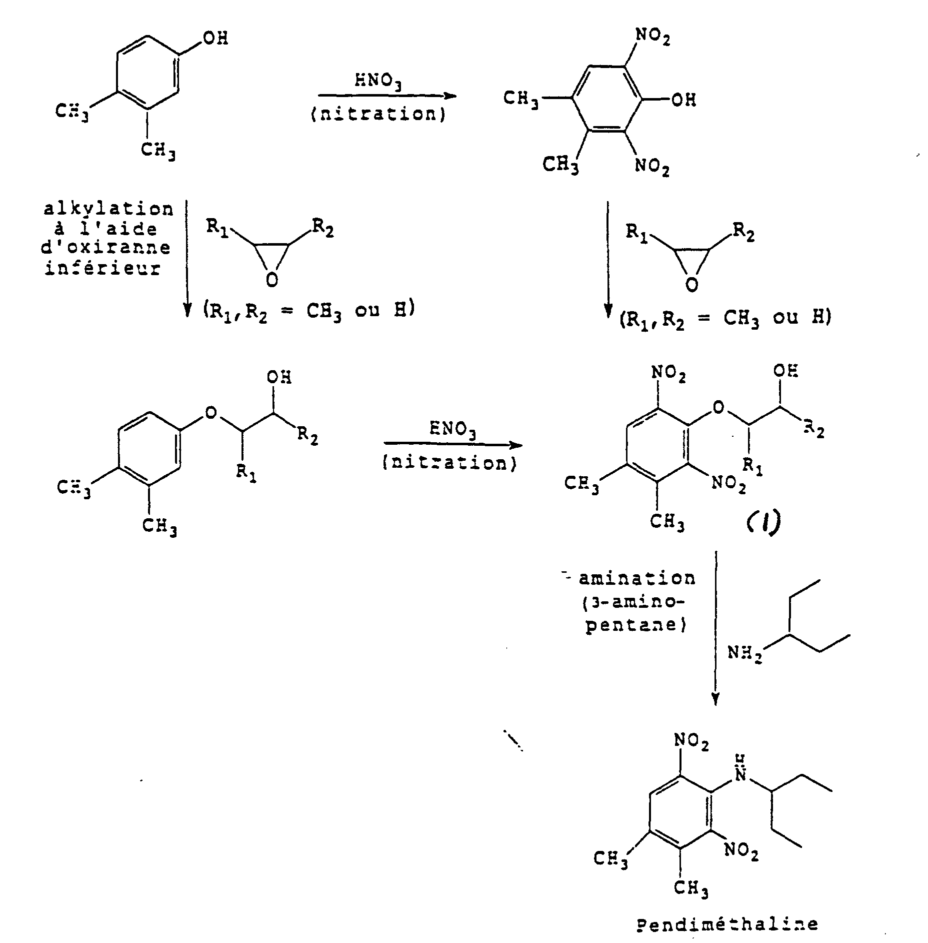

### EXEMPLE 1

### Préparation d'un α-phénoxy-alcanol de formule (I).

Dans un autoclave de 100 ml, on charge 4 g (18,8 mmol) de 2,6-dinitro-3,4-diméthylphénol à 99,9%; on introduit 2,21 · g (38 mmol) de 1,2-époxypropane à 99% et 3 gouttes de catalyseur basique. On ferme l'appareil et on comprime à 3 bars de pression d'azote. Puis on porte la température à 100°C; la réaction est suivie par chromatographie sur couche mince; après 3 heures de maintien à 100°C, le taux de phénol résiduel est inférieur à 1% et la réaction est arrêtée.

On refroidit le réacteur à la température ambiante, on le décomprime et on recueille 5,10 g (100% de rendement) d'un produit rouge brun constitué
- à 70% de 1-(2,6-dinitro-3,4-diméthyl-phénoxy)-2-propanol et
- à 30% de 2-(2,6-dinitro-3,4-diméthyl-phénoxy)-1-propanol.

Ces deux isomères ne sont pas séparés car ils sont tous deux précurseurs de la pendiméthaline.

### EXEMPLE 2

### Utilisation d'un α-phénoxy-alcanol de formule (I) dans la préparation de la pendiméthaline.

Dans un ballon tricol de 50 ml, équipé d'une agitation, d'un thermomètre, d'un condenseur à reflux et d'une ampoule de coulée, on charge 26,0 g (97,0 mmol) du mélange des deux α-(2,6-dinitro-3,4-diméthyl-phénoxy)-propanol préparé selon l'exemple 1, puis on introduit en 10 minutes 59,5 g (687 mmol) de 3-amino-pentane.

On laisse réagir pendant 14 heures à 90°C sous reflux; l'avancement de la réaction est suivi par chromatographie sur couche mince; après disparition de l'éther, la réaction est stoppée.

On distille à pression atmosphérique l'excès d'amino-3-pentane n'ayant pas réagi, qui passe à 90°C sous pression atmosphérique et qui, une fois récupéré, peut être recyclé pour une opération d'amination ultérieure.

On charge alors 100 ml de xylène puis, après deux lavages à chaud avec 50 ml d'eau, on distille le xylène sous pression réduite.

Après évaporation totale du xylène, on recueille 26,4 g (soit 95% de rendement) de pendiméthaline à 97% de pureté.

### EXEMPLE 3

### Utilisation d'un α-phénoxy-alcanol de formule (I) dans la préparation de la pendiméthaline.

Dans un autoclave inox de 100 ml, équipé d'une agitation et d'un thermomètre, on charge 25,0 g (92,5 mmol) du mélange des deux α-(2,6-dinitro-3,4-diméthyl-phénoxy)-propanol préparés selon l'exemple 1, puis on introduit en 30 minutes à 90°C une quantité de 40,3 g (462 mmol) de 3-amino-pentane.

On laisse réagir sous une pression de 2 atmosphères pendant 3 heures à 90°C, puis pendant 2 heures à 100°C, et enfin pendant 3 heures à 130°C; l'avancement de la réaction est suivi par chromatographie sur couche mince; après disparition de l'éther, la réaction est stoppée.

On dépressurise le réacteur puis on sépare par distillation à pression atmosphérique l'excès d'amino-3-pentane n'ayant pas réagi; celui-ci passe à 90°C et peut être recyclé dans une opération ultérieure.

On charge alors 100 ml de xylène dans l'enceinte réactionnelle, on lave deux fois à chaud avec 50 ml d'eau, puis on distille le xylène sous pression réduite.

Après évaporation totale du xylène, on recueille 26,4 g (soit 98% de rendement) de pendiméthaline à 96,4% de pureté.

## Revendications

1. α-phénoxy-alcanols représentés par la formule générale 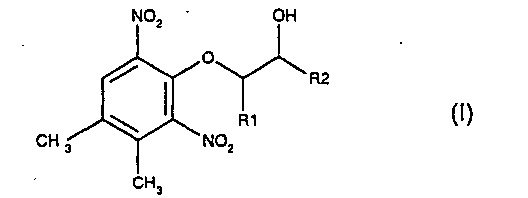 dans laquelle R₁ et R₂, qui sont identiques ou différents l'un de l'autre, représentent un atome d'hydrogène ou le radical CH₃,
et leurs stéroisomères de conformation R,S lorsqu'ils comportent au moins un carbone asymétrique.

2. Procédé de préparation des α-phénoxy-alcanols selon la revendication 1, **caractérisé par le fait que** l'on fait réagir le 2,6-dinitro-3,4-diméthylphénol avec un oxiranne inférieur choisi dans le groupe comprenant l'oxyde d'éthylène, l'oxyde propylène et le 2,3-époxybutane.

3. Procédé selon la revendication 2, **caractérisé par le fait qu'**il est mis en oeuvre à une température choisie dans le domaine de -40 à 300°C, de préférence de -20 à 200°C et plus préférentiellement encore de -10 à 150°C.

4. Procédé selon l'une des revendications 2 et 3, **caractérisé par le fait qu'**il est mis en oeuvre sous une pression totale de 0,98692 à 98,692 bars, plus préférentiellement de 0,98692 à 24,67 bars et, plus préférentiellement encore, sous une pression totale inférieure à 9,8692 bars.

5. Procédé selon l'une des revendications 2 à 4, **caractérisé par le fait que** les quantités molaires d'oxiranne mises en oeuvre par rapport au phénol engagé sont dans des rapports de 1 à 100, de préférence de 1 à 20 et, plus préférentiellement encore, de 1 à 5.

6. Procédé selon l'une des revendications 2 à 5, **caractérisé par le fait qu'**il est mis en oeuvre en présence d'un catalyseur basique choisi dans le groupe comprenant les amines tertiaires aliphatiques notamment du groupe comprenant la triméthylamine, la méthyldiéthylamine et la triéthylamine, les anilines tertiaires dont notamment la diméthylaniline et la diéthylaniline, les amines cycliques dont notamment la pyridine et les imidazoles.

7. Procédé selon la revendication 6, **caractérisé par le fait que** le catalyseur est mis en oeuvre à raison de 10 ppm à 10000 ppm et, de préférence, à raison de 50 à 1000 ppm.

8. Procédé selon l'une des revendications 2 à 7, **caractérisé par le fait qu'**il est mis en oeuvre en l'absence de solvant.

9. Procédé selon l'une des revendications 2 à 7, **caractérisé par le fait qu'**il est mis en oeuvre au sein d'un solvant organique inerte choisi dans le groupe comprenant les essences aliphatiques dont notamment l'hexane, l'heptane, l'octane, le nonane et le dodécane, les dérivés chlorés dont notamment le dichlorométhane, le 1,2-dichloroéthane, le chloroforme, le trichloréthylène et le tétrachloréthylène, les dialkyl-éthers dont notamment les diéthyl- et dipropyl-éthers ainsi que les dibutyl-éthers, et de préférence les solvants aromatiques dont notamment le benzène, le toluène et le xylène.

10. Application des α-phénoxy-alcanols selon la revendication 1 à la préparation de la pendiméthaline.

## Patentansprüche

1. α-Phenoxyalkanole der allgemeinen Formel 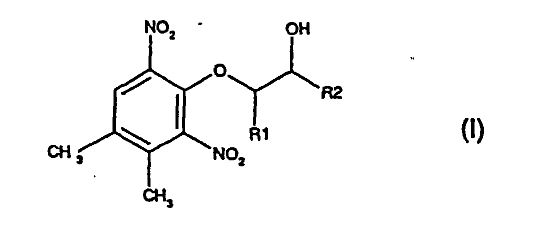 in der R₁ und R₂, die gleich oder verschieden sind, ein Wasserstoffatom oder den Rest CH₃ darstellen, und ihre Steroisomere der Form R,S, wenn sie mindestens einen asymmetrischen Kohlenstoff enthalten.

2. Verfahren zur Herstellung der a-Phenoxyalkanole nach Anspruch 1, **dadurch gekennzeichnet, dass** man 2,6-Dinitro-3,4-dimethylphenol mit einem niederen Oxiran reagieren lässt, das aus der Gruppe ausgewählt ist, die Ethylenoxid, Propylenoxid und 2,3-Epoxybutan umfasst.

3. Verfahren nach Anspruch 2, **dadurch gekennzeichnet, dass** es bei einer Temperatur durchgeführt wird, die im Bereich von -40 bis 300°C, vorzugsweise von -20 bis 200°C und noch bevorzugter von -10 bis 150°C ausgewählt wird.

4. Verfahren nach einem der Ansprüche 2 und 3, **dadurch gekennzeichnet, dass** es unter einem Gesamtdruck von 0,98692 bis 98,692 bar, vorzugsweise 0,98692 bis 24,67 bar und noch bevorzugter unter einem Gesamtdruck von weniger als 9,8692 bar durchgeführt wird.

5. Verfahren nach einem der Ansprüche 2 bis 4, **dadurch gekennzeichnet, dass** die verwendeten molaren Mengen von Oxiran im Verhältnis zu dem eingesetzten Phenol in Verhältnissen von 1 bis 100, vorzugsweise von 1 bis 20 und noch bevorzugter von 1 bis 5 liegen.

6. Verfahren nach einem der Ansprüche 2 bis 5, **dadurch gekennzeichnet, dass** es in Gegenwart eines basischen Katalysators durchgeführt wird, der aus der Gruppe ausgewählt wird, die die tertiären aliphatischen Amine insbesondere der aus Trimethylamin, Methyldiethylamin und Triethylamin bestehenden Gruppe, die tertiären Aniline, insbesondere Dimethylanilin und Diethylanilin, die zyklischen Amine, insbesondere Pyridin und die Imidazole, umfasst.

7. Verfahren nach Anspruch 6, **dadurch gekennzeichnet, dass** der Katalysator in einer Menge von 10 ppm bis 10000 ppm und vorzugsweise in einer Menge von 50 bis 1000 ppm eingesetzt wird.

8. Verfahren nach einem der Ansprüche 2 bis 7, **dadurch gekennzeichnet, dass** es ohne Lösungsmittel durchgeführt wird.

9. Verfahren nach einem der Ansprüche 2 bis 7, **dadurch gekennzeichnet, dass** es in einem neutralen organischen Lösungsmittel durchgeführt wird, das aus der Gruppe ausgewählt wird, die die aliphatischen Öle, insbesondere Hexan, Heptan, Octan, Nonan und Dodecan, die Chlorderivate, insbesondere, Dichlormethan, 1,2-Dichlorethan, Chloroform, Trichlorethan und Tetrachlorethylen, die Dialkylether, insbesondere die Diehtyl- und Dipropylether sowie die Dibutylether, und vorzugsweise die aromatischen Lösungsmittel, insbesondere Benzol, Toluol und Xylol, umfasst.

10. Verwendung der a-Phenoxyalkanole nach Anspruch 1 zur Herstellung von Pendimethalin.

## Claims

1. α-phenoxy-alkanols represented by the general formula 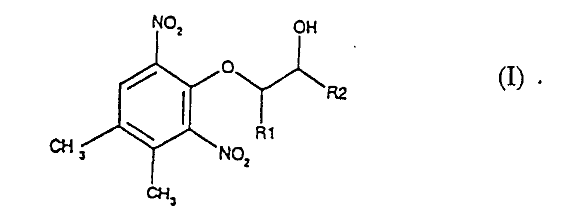 in which R₁ and R₂, which are identical or different, represent a hydrogen atom or the CH₃ radical,
and their stereoisomers of R,S conformation when they contain at least one asymmetric carbon.

2. A process for preparation of the α-phenoxy-alkanols according to claim 1, **characterized in that** 2,6-dinitro-3,4-dimethylphenol is reacted with a lower oxirane selected from the group comprising ethylene oxide, propylene oxide and 2,3-epoxybutane.

3. A process according to claim 2, **characterized in that** it is carried out at a temperature chosen in the range from -40 to 300°C, preferably from -20 to 200°C and even more preferably from -10 to 150°C.

4. A process according to one of the claims 2 and 3, **characterized in that** it is carried out under a total pressure of 0,98692 to 98,692 bars, more preferably from 0,98692 to 24,67 bars and, even more preferably, under a total pressure of less than 9,8692 bars.

5. A process according to one of the claims 2 to 4, **characterized in that** the molar amounts of oxirane employed relative to the phenol used are in ratios of from 1 to 100, preferably from 1 to 20 and, even more preferably, from 1 to 5.

6. A process according to one of the claims 2 to 5, **characterized in that** it is carried out in the presence of a basic catalyst selected from the group comprising the aliphatic tertiary amines and especially from the group comprising trimethylamine, methyldiethylamine and triethylamine, the tertiary anilines and especially dimethylaniline and diethylaniline, the cyclic amines and especially pyridine and the imidazoles.

7. A process according to claim 6, **characterized in that** the catalyst is employed at the rate of 10 ppm to 10000 ppm and preferably at the rate of 50 to 1000 ppm.

8. A process according to one of the claims 2 to 7, **characterized in that** it is carried out in the absence of solvent.

9. A process according to one of the claims 2 to 7, **characterized in that** it is carried out in an inert organic solvent selected from the group comprising the aliphatic hydrocarbons and especially hexane, heptane, octane, nonane and dodecane, the chlorinated derivatives and especially dichloromethane, 1,2-dichloroethane, chloroform, trichloroethylene and tetrachloroethylene, the dialkyl-ethers and especially diethyl- and dipropyl-ethers as well as the dibutyl-ethers, and preferably the aromatic solvents and especially benzene, toluene and xylene.

10. Use of the α-phenoxy-alkanols according to claim 1 in the preparation of pendimethalin.
